(19) Europäisches Patentamt
European Patent Office
Office européen des brevets

(11) **EP 3 028 691 B1**

(12) **EUROPEAN PATENT SPECIFICATION**

(45) Date of publication and mention
of the grant of the patent:
**03.03.2021 Bulletin 2021/09**

(21) Application number: **14831417.2**

(22) Date of filing: **20.06.2014**

(51) Int Cl.:
*A61K 8/39* *(2006.01)*     *A61K 8/68* *(2006.01)*
*A61K 8/41* *(2006.01)*     *A61K 8/42* *(2006.01)*
*A61K 8/44* *(2006.01)*     *A61K 8/34* *(2006.01)*
*A61Q 5/02* *(2006.01)*     *A61Q 5/12* *(2006.01)*

(86) International application number:
**PCT/JP2014/066465**

(87) International publication number:
**WO 2015/015947 (05.02.2015 Gazette 2015/05)**

(54) **CERAMIDE DISPERSION COMPOSITION**

CERAMID-DISPERSIONSZUSAMMENSETZUNG

COMPOSITION DE DISPERSION DE CÉRAMIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.08.2013 JP 2013161737**

(43) Date of publication of application:
**08.06.2016 Bulletin 2016/23**

(73) Proprietor: **FUJIFILM Corporation
Minato-ku
Tokyo 106-8620 (JP)**

(72) Inventors:
• **KITAOKA, Hiroyuki
Ashigarakami-gun
Kanagawa 258-8577 (JP)**
• **MORI, Mikinaga
Ashigarakami-gun
Kanagawa 258-8577 (JP)**
• **ARAKAWA, Jun
Ashigarakami-gun
Kanagawa 258-8577 (JP)**

(74) Representative: **Klunker IP
Patentanwälte PartG mbB
Destouchesstraße 68
80796 München (DE)**

(56) References cited:
**EP-A1- 2 452 668     JP-A- 2003 113 393
JP-A- 2003 300 842     JP-A- 2003 300 842
JP-A- 2005 206 573     JP-A- 2007 015 986
JP-A- 2012 171 946     US-A1- 2005 287 095**

**Description**

**BACKGROUND OF THE INVENTION**

1. Field of the Invention

The present invention relates to a ceramide dispersion composition.

2. Description of the Related Art

Ceramides are in the stratum corneum of skin and play an important role in retaining moisture by constructing a lipid barrier necessary for retaining moisture. Ceramides in the stratum corneum are generated as a result of the decomposition of cerebrosides due to an enzyme called cerebrosidase. There are six different types of ceramide in the human skin, and they perform different functions. In anticipation of the functions of ceramides being expressed, various products formulated with ceramides are being developed.

However, a ceramide is a substance having high crystallinity, exhibits low solubility in oil, and is precipitated in the form of crystals at low temperature. Therefore, it is difficult to formulate the ceramide with excellent stability.

As a technique for stably formulating the ceramide in the form of dispersed particles having a micro-particle size, a method of formulating a specific fatty acid, a specific surfactant, a specific phospholipid, and the like with ceramides is known (for example, see JP2010-90092A, WO2009/145299A, JP2008-69108A, and JP2007-1950A).

JP 2003-300842 A discloses a cosmetic capable of stably formulating ceramides independently from shapes, excellent in preservation stability. This cosmetic comprises (a) the ceramides and (b) a specific amidoalcohol.

EP 2452668 A1 teaches an emulsion composition which contains (A) 0.001 to 10 wt.% of an organic compound having two or more hydroxyl groups, an inorganic value of 220 to 450, and an organic value of 300 to 1,000; (B) 0.001 to 10 wt.% of an organic compound having one hydroxyl group, an inorganic value of 100 to 200, and an organic value of 280 to 700; (C) 0.001 to 10 wt.% of a compound represented by formula (2); (D) 0.00012 to 10 wt.% of at least one compound selected from the group consisting of a nonionic surfactant having a polyoxyethylene group and an HLB of 10 or higher, an ionic surfactant, and a sphingosine salt; (E) 0.003 to 15 wt.% of at least one compound selected from the group consisting of a sugar alcohol selected from the group consisting of erythritol, threitol, xylitol, and mannitol, a disaccharide, and a trisaccharide; and (F) water.

US 2005/287095 A1 discloses a vesicle dispersion comprising (A) sucrose fatty acid ester, (B) a sphingosine and/or its derivative, and (C) an aqueous component and a cosmetic composition comprising the vesicle dispersion. The vesicle dispersion can stably contain sphingosines such as a ceramide, excelling in a moisturizing effect of the skin, and the cosmetic composition comprising the vesicle dispersion exhibits an excellent moisturizing effect, storage stability, and the like.

JP 2003-113393 A teaches a ceramide dispersion capable of being used in various fields, such as pharmaceutical, cosmetic, and dietary fields, by changing the ceramide slightly soluble in water into a water-soluble composition, scarcely causing isolation nor precipitation of the ceramide even when stored for a long period, capable of being kept in a uniformly and stably emulsified, dispersed or solubilized state, having excellent acid-resisting, salt-resisting and heat-resisting properties which are necessary when added to food and drink, capable of preventing the ceramide from depositing, precipitating, and floating in the food and drink and a cosmetic to which the dispersion is added, and having excellent transparency. This ceramide dispersion is formed by dispersing the ceramide (A) in a water-based solvent, wherein an ester (B1) of a 8-10C fatty acid of a polyglycerol and another ester (B2) of a 12-18C fatty acid of the polyglycerol are combined to be used.

**SUMMARY OF THE INVENTION**

Because a hair-forming protein is acidic, a hair conditioner, a hair treatment, a rinse, and the like are designed to be acidic in some cases, and it is desirable to use ceramides under acidic conditions. Furthermore, considering permeability with respect to the skin and the like, it is desirable that the dispersed particles have a small particle size. However, it is known that under acidic pH conditions, a ceramide dispersion composition containing fine particles which is stable over a long period of time is not easily obtained.

An object of the present invention is to provide a ceramide dispersion composition which contains fine dispersed particles and exhibits excellent temporal stability under acidic conditions.

The present invention, which is defined in the appended claims, is as below.

[1] A ceramide dispersion composition having a pH of equal to or less than 6.5, comprising: a natural ceramide; a polyol; a first surfactant which is a nonionic surfactant; and a second surfactant which is at least one selected from the group consisting of a cationic surfactant and an amphoteric surfactant excluding phospholipids, wherein the first surfactant comprises a polyglyceryl fatty acid.

[2] The ceramide dispersion composition according to [1], wherein the second surfactant comprises at least one component selected from the group consisting of an amino acid derivative, a quaternary ammonium salt, and an amidoamine derivative.

[3] The ceramide dispersion composition according to [1] or [2], wherein the second surfactant comprises an arginine derivative.

[4] The ceramide dispersion composition according to any one of [1] to [3], wherein an oil phase component which comprises at least the natural ceramide is in a form of particles having an average particle size equal to or less than 100 nm and being dispersed in an aqueous phase component.

[5] The ceramide dispersion composition according to any one of [1] to [4], wherein the polyol comprises glycerin.

[6] The ceramide dispersion composition according to any one of [1] to [5], wherein the natural ceramide comprises ceramide 2.

[7] The ceramide dispersion composition according to any one of [1] to [6], wherein a content of the first surfactant is from 0.2% by mass to 2.5% by mass with respect to a total mass of the ceramide dispersion composition.

[8] The ceramide dispersion composition according to any one of [1] to [7], wherein a content of the polyol is from 2.0% by mass to 50.0% by mass with respect to a total mass of the ceramide dispersion composition.

[9] The ceramide dispersion composition according to any one of [1] to [8], wherein a content of the second surfactant is from 0.05% by mass to 8.0% by mass with respect to a total mass of the ceramide dispersion composition.

[10] The ceramide dispersion composition according to any one of [1] to [9], wherein a total content of the first surfactant and the second surfactant is from 0.5% by mass to 10.0% by mass with respect to a total mass of the ceramide dispersion composition.

[11] The ceramide dispersion composition according to any one of [1] to [10], wherein a total content of the first surfactant and the second surfactant is from 0.7% by mass to 5.0% by mass with respect to a total mass of the ceramide dispersion composition.

[12] The ceramide dispersion composition according to any one of [1] to [11], wherein a content of the natural ceramide is from 0.1% by mass to 5.0% by mass with respect to a total mass of the ceramide dispersion composition.

[13] A hair cosmetic comprising the ceramide dispersion composition according to any one of [1] to [12].

According to the present invention, it is possible to provide a ceramide dispersion composition which contains fine dispersed particles and exhibits excellent temporal stability under acidic conditions.

## DESCRIPTION OF THE PREFERRED EMBODIMENTS

The ceramide dispersion composition of the present invention contains a natural ceramide, a polyol, a first surfactant which is a nonionic surfactant which comprises a polyglyceryl fatty acid, and a second surfactant which is at least one selected from the group consisting of a cationic surfactant and an amphoteric surfactant excluding phospholipids. Furthermore, the ceramide dispersion composition of the present invention has a pH of equal to or less than 6.5.

By adopting the constitution described above, the ceramide dispersion composition of the present invention contains fine dispersed particles and exhibits excellent temporal stability under acidic conditions. That is, by adding a polyol and a combination of specific first and second surfactants to a ceramide composition containing a natural ceramide and placing the ceramide dispersion composition under acidic conditions of a pH of equal to or less than 6.5, it is possible to prevent the dispersed particles from expanding with the passage of time under the acidic conditions. If phospholipids are present in the amphoteric surfactant, fine dispersed particles are not obtained, and the effect of preventing expansion of the dispersed particles becomes insufficient. Presumably, because phospholipids exhibit strong hydrophobicity in an acidic region and function poorly as a dispersing agent, the presence of phospholipids results in failing to obtain fine dispersed particles and makes the effect of preventing expansion of the dispersed particles insufficient. However, the present invention is not limited to this presumption.

In the present specification, a term "step" includes not only an independent step but also steps that cannot be clearly differentiated from other steps as long as an intended purpose thereof is accomplished.

In the present specification, a range of numerical values represented by using "to" is a range including numerical values listed before and after "to" as a minimum value and a maximum value respectively.

In the present specification, unless otherwise specified, when there is a plurality of substances corresponding to each of the components in the composition, the amount of each of the components in the composition means the total amount of the plurality of substances in the composition.

In the present invention, a "aqueous phase" is used as a term which is in contrast to an "oil phase" regardless of the type of a solvent.

Hereinafter, the present invention will be described.

<Natural ceramide>

The ceramide dispersion composition of the present invention contains at least a natural ceramide. The natural ceramide is contained in the ceramide dispersion composition, in the form of dispersed particles that are dispersed in an aqueous phase in the form of an oil phase (oil droplets).

In the present invention, the natural ceramide refers to ceramide having the same structure as ceramide present in the stratum corneum of human skin. In a more preferred embodiment, the natural ceramide does not include glycosphingolipids and has three or more hydroxyl groups in a molecular structure thereof.

Ceramide called a natural ceramide having a general structure may be a natural substance (extract), ceramide obtained by a microbial fermentation method, a synthetic substance, or ceramide derived from animals.

As the ceramide, an optically active isomer of a natural ceramide (D(-) enantiomer) is used. Furthermore, if necessary, an optically active isomer of a non-natural ceramide (L(+) enantiomer) may be used. In addition, a mixture of an optically active isomer of a natural ceramide and an optically active isomer of a non-natural ceramide may also be used. The relative configuration of ceramide may be a configuration of a natural ceramide, a configuration of a non-natural ceramide, or a configuration of a mixture of a natural ceramide and a non-natural ceramide.

Examples of the natural ceramide which can be preferably used in the present invention include ceramide 1, ceramide 9, ceramide 4, ceramide 2, ceramide 3, ceramide 5, ceramide 6, ceramide 7, ceramide 8, ceramide 3B, and the like. It is preferable that the ceramide dispersion composition of the present invention contains, as the natural ceramide, ceramide 2 which is known as a main component of ceramide in hair among the above ceramides.

Commercial products are available as the natural ceramide, and examples thereof include Ceramide I, Ceramide III, Ceramide IIIA, Ceramide IIIB, Ceramide IIIC, and Ceramide VI (all manufactured by Evonik Industries AG); Ceramide TIC-001 (manufactured by Takasago International Corporation); CERAMIDE II (manufactured by Quest International); DS-Ceramide VI, DS-CLA-Phytoceramide, C6-Phytoceramide, and DS-Ceramide Y3S (manufactured by Doosan Corporation); CERAMIDE 2 (manufactured by Sederma); and the like.

One kind of natural ceramide may be used singly, or two or more kinds thereof may be used concurrently.

As the natural ceramide in the ceramide dispersion composition, according to the purpose, it is possible to use those modified by introducing a double bond into a molecule thereof so as to impart solubility or by introducing a hydrophobic group thereinto so as to impart permeability.

From the viewpoint of formulating the ceramide as an active component at high concentration and from the viewpoint of the dispersion stability, the content of the natural ceramide is preferably 1% by mass to 100% by mass, more preferably 3% by mass to 80% by mass, and even more preferably 10% by mass to 70% by mass, with respect to the total mass of the oil phase components forming the oil phase of the ceramide dispersion composition.

Furthermore, from the viewpoint of formulating the ceramide as an active component at high concentration and from the viewpoint of the dispersion stability, the content of the natural ceramide in the ceramide dispersion composition is preferably 0.02% by mass to 10% by mass, more preferably 0.1% by mass to 5% by mass, and even more preferably 0.2% by mass to 4% by mass, with respect to the total mass of the ceramide dispersion composition.

<Polyol>

The ceramide dispersion composition contains a polyol. Because the ceramide dispersion composition contains the polyol, the dispersed particles contained in the ceramide dispersion composition can be further atomized, and the ceramide dispersion composition can retain the dispersed particles having micro-particle size that are stable over a long period time.

As the polyol, any polyol can be used without particular limitation as long as it is an alcohol having two or more hydroxyl groups. Examples of the polyol include glycerin, diglycerin, triglycerin, polyglycerin, 3-methyl-1,3-butanediol, 1,3-butylene glycol (1,3-BG), isoprene glycol, polyethylene glycol, 1,2-pentanediol, 1,2-hexanediol, propylene glycol, dipropylene glycol, polypropylene glycol, ethylene glycol, diethylene glycol, neopentyl glycol, maltitol, reduced sugar syrup, sucrose, lactitol, palatinit, erythritol, sorbitol, mannitol, xylitol, xylose, glucose, lactose, mannose, maltose, galactose, fructose, inositol, pentaerythritol, maltotriose, sorbitan, trehalose, sugar obtained by amylolysis, a reduced sugar alcohol obtained by amylolysis, and the like. Among these, in view of the temporal stability, a polyol having 5 or less carbon atoms in total is preferable as the polyol, and a polyol having 3 or less carbon atoms in total is more preferable as the polyol. Particularly, from the viewpoint of the temporal stability, the polyol is preferably glycerin, propanediol, or the like, and more preferably glycerin.

From the viewpoint of the dispersibility and the temporal stability, the content of the polyol is preferably 2.0% by mass to 50.0% by mass, more preferably 5.0% by mass to 45.0% by mass, and even more preferably 10.0% by mass to 40.0% by mass, with respect to the total mass of the ceramide dispersion composition. If the content of the polyol is equal to or greater than 2.0% by mass with respect to the total mass of the ceramide dispersion composition, the dispersibility of the ceramide dispersion composition tends to become excellent, and the dispersed particles tend to be atomized. If the content of the polyol is equal to or less than 50.0% by mass, the temporal stability tends to become excellent.

<First surfactant>

The first surfactant in the ceramide dispersion composition is a nonionic surfactant which comprises a polyglyceryl fatty acid. Examples of the nonionic surfactant include a monocleryl fatty acid, a monoglyceryl organic acid, a polyglyceryl fatty acid, a propylene glycol fatty acid ester, a polyglycerin condensed ricinoleic acid ester, a sorbitan fatty acid ester, a sucrose fatty acid ester, a polyoxyethylene sorbitan fatty acid ester, and the like. One kind of the first surfactant may be used singly, or two or more kinds thereof may be used in combination.

From the viewpoint of atomizing the dispersed particles, the content of the first surfactant is preferably 0.2% by mass to 2.5% by mass, more preferably 0.4% by mass to 2.0% by mass, and even more preferably 0.7% by mass to 1.5% by mass, with respect to the total mass of the ceramide dispersion composition. If the content of the first surfactant is equal to or greater than 0.2% by mass with respect to the total mass of the ceramide dispersion composition, the dispersibility of the ceramide dispersion composition tends to be improved, and the dispersed particles tend to be further atomized. If the content of the first surfactant is equal to or less than 2.5% by mass, the temporal stability of the ceramide dispersion composition tends to be further improved.

From the viewpoint of the dispersion stability, among the above surfactants, a polyglyceryl fatty acid is preferable as the first surfactant, and a polyglyceryl fatty acid having an HLB of equal to or greater than 10 and equal to or less than 16 is more preferable as the first surfactant. The polyglyceryl fatty acid having an HLB of equal to or greater than 10 and equal to or less than 16 is preferable since the surfactant can greatly reduce the interfacial tension between an oil phase and an aqueous phase, and thus the dispersed particles can be atomized.

An HLB means the hydrophilic-hydrophobic balance used generally in the field of surfactants. For calculating the HLB, it is possible to use a generally used calculation formula such as a Kawakami's formula. In the present invention, the following Kawakami's formula is adopted as a calculation formula for the HLB.

$$\mathrm{HLB} = 7 + 11.7 \log(\mathrm{Mw/Mo})$$

Mw represents the molecular weight of a hydrophilic group, and Mo represents the molecular weight of a hydrophobic group.

The numerical values of HLB listed in a catalog or the like may also be used. As is evident from the above formula, by using the additivity of the HLB, a surfactant having any HLB value can be obtained.

As one of the polyglyceryl fatty acids, an ester of at least a polyglycerin having an average polymerization degree of 10 and a fatty acid having 8 to 18 carbon atoms selected from the group consisting of, for example, caprylic acid, capric acid, lauric acid, myristic acid, palmitic acid, stearic acid, oleic acid, and linoleic acid is particularly preferable.

Preferred examples of the polyglyceryl fatty acid which is an ester of a polyglycerin having an average polymerization degree of 10 and a fatty acid having 8 to 18 carbon atoms include hexaglyceryl oleate, hexaglyceryl palmitate, hexaglyceryl myristate, hexaglyceryl laurate, decaglyceryl linoleate, decaglyceryl oleate, decaglyceryl stearate, decaglyceryl palmitate, decaglyceryl myristate, decaglyceryl laurate, and the like. All of these polyglyceryl fatty acids have an HLB of equal to or greater than 10 and equal to or less than 16.

Among the above, as the polyglyceryl fatty acid, decaglyceryl monolinoleate (HLB = 12), decaglyceryl monooleate (HLB = 12), decaglyceryl monostearate (HLB = 12), decaglyceryl monopalmitate (HLB = 13), decaglyceryl monomyristate (HLB = 14), decaglyceryl monolaurate (HLB = 16), and the like are more preferable. One kind of these polyglyceryl fatty acids can be used singly, or two or more kinds thereof can be used.

In the present invention, the first surfactant may be a combination of one or more kinds of surfactants selected from polyglyceryl fatty acids having an HLB of equal to or greater than 10 and equal to or less than 16 and one or more kinds of surfactants selected from polyglyceryl fatty acids having an HLB of equal to or greater than 5 and equal to or less than 15 that have a molecular structure different from that of the aforementioned polyglyceryl fatty acids. The polyglyceryl fatty acids having an HLB of equal to or greater than 5 and equal to or less than 15 may be polyglyceryl fatty acids included in the polyglyceryl fatty acids described above, or may be polyglyceryl fatty acids other than these.

<Second surfactant>

The second surfactant in the ceramide dispersion composition is at least one kind of surfactant selected from the group consisting of an amphoteric surfactant and a cationic surfactant. Here, the amphoteric surfactant in the second surfactant does not include phospholipids. By combining at least one kind of surfactant, which is selected from the group consisting of a cationic surfactant and an amphoteric surfactant excluding phospholipids, with a nonionic surfactant as the first surfactant, the dispersed particles containing the polyol and the natural ceramide can remain as fine particles. When a phospholipid is used as the amphoteric surfactant in the second surfactant, even if the amphoteric surfactant is combined with the first surfactant, the effect of making the dispersed particles containing the natural ceramide remain as fine particles is not obtained.

The second surfactant may be embodied as surfactant which is composed solely of an amphoteric surfactant, surfactant which is composed solely of a cationic surfactant, or surfactants which are a combination of an amphoteric surfactant and a cationic surfactant. In these embodiments, one kind of each of the amphoteric surfactant and the cationic surfactant may be used singly, or two or more kinds of each of the amphoteric surfactant and the cationic surfactant may be used in combination. In a case where the second surfactant is embodied as a combination of an amphoteric surfactant and a cationic surfactant, the embodiment of the second surfactant includes a combination of one kind of amphoteric surfactant and one kind of cationic surfactant, a combination of one kind of amphoteric surfactant and two or more kinds of cationic surfactant, a combination of two or more kinds of amphoteric surfactant and one kind of cationic surfactant, and a combination of two or more kinds of amphoteric surfactant and two or more kinds of cationic surfactant.

Examples of the amphoteric surfactant as the second surfactant include an amino acid type, a betaine type, a sulfuric acid ester salt type, a sulfonic acid type, and the like. In view of atomizing the dispersed particles and the temporal stability of the ceramide dispersion composition, an amphoteric surfactant selected from the group consisting of an amino acid type and a betaine type is preferable. Examples of the amino acid type and betaine type amphoteric surfactants include an imidazoline derivative such as sodium 2-undecyl-N-carboxymethyl-N-hydroxyethylimidazolinium betaine, a 2-cocoyl-2-imidazoliniumhydroxide1-carboxyethyloxy disodium salt, or 2-heptadecyl-N-carboxymethyl-N-hydroxyethylimidazolinium betaine; a betaine derivative such as lauryldimethyl aminoacetic acid betaine, cocamidopropyl betaine, oleamidopropyl betaine, lauramidopropyl betaine, coco-betaine, oleyl betaine, lauryl betaine, cetyl betaine, sulfobetaine, or coconut oil fatty acid amidopropyl betaine; an amino acid derivative such as an alkyloxyhydroxypropyl amino acid salt; and the like.

Examples of the cationic surfactant as the second surfactant include a quaternary ammonium salt type, an amine salt type, a pyridinium salt type, and the like. Specifically, examples thereof include an alkyltrimethyl ammonium chloride such as behentrimonium chloride, steartrimonium chloride, cetrimonium chloride, or lauryltrimonium chloride; an alkyltrimethyl ammonium bromide such as stearyltrimonium bromide; a dialkyldimethyl ammonium chloride such as distearyldimonium chloride or dicocodimonium chloride; a fatty acid amidoamine and a salt thereof such as stearamidopropyl dimethylamine or stearamidoethyl diethylamine; an alkylether amine and a salt or a quaternary salt thereof such as stearoxypropyl dimethylamine; a fatty acid amide-type quaternary ammonium salt such as ethyl sulfate long-chain branched fatty acid (12 to 31) aminopropylethyl dimethyl ammonium; a fatty acid amide-type quaternary ammonium salt such as ethyl sulfate lanolin fatty acid aminopropyl ethyldimethyl ammonium; a polyoxyethylene alkylamine and a salt or a quaternary salt thereof; an alkylamine salt; a fatty acid amide guanidium salt; an alkylether ammonium salt; an alkyltrialkylene glycol ammonium salt; a benzalkonium salt; a benzethonium salt; a pyridinium salt such as cetylpyridnium chloride; an imidazolium salt; an alkyl isoquinolinium salt; a dialkyl morpholinium salt; a polyamine fatty acid derivative; and a silicone-based cationic surfactant such as an amino-modified silicone such as aminopropyl dimethicone and amodimethicone, cation-modified silicone, cation- and polyether-modified silicone, or amino- and polyether-modified silicone.

In view of the temporal stability of the ceramide dispersion composition, the second surfactant is preferably at least one kind of surfactant selected from the group consisting of an amino acid derivative; a quaternary ammonium salt such as an alkyltrimethyl ammonium chloride, an alkyltrimethyl ammonium bromide, or a dialkyldimethyl ammonium chloride; and an amidoamine derivative such as a fatty acid amidoamine and a salt thereof. In view of atomizing the dispersed particles and the temporal stability of the ceramide dispersion composition, the second surfactant is preferably an amino acid derivative.

Examples of the amino acid derivative preferable as the second surfactant include an alkyloxyhydroxypropyl amino acid salt. Examples of the amino acid in the alkyloxyhydroxypropyl amino acid salt include arginine, alanine, leucine, and the like. Examples of the alkyl group in the alkyloxyhydroxypropyl amino acid salt include an alkyl group having 6 to 22 carbon atoms. In view of atomizing the dispersed particles and the temporal stability of the ceramide dispersion composition, for example, an alkyl group having 8 to 18 carbon atoms is preferable as the alkyl group. The amino acid derivative as the second surfactant is preferably an arginine derivative, and specific examples thereof include an alkyloxyhydroxypropyl arginine salt such as N-[3-alkyl(12,14)oxy-2-hydroxypropyl]-L-arginine hydrochloride.

From the viewpoint of atomizing the dispersed particles and the temporal stability of the ceramide dispersion composition, the content of the second surfactant is preferably 0.05% by mass to 8.0% by mass, more preferably 0.1% by mass to 5.0% by mass, and even more preferably 0.2% by mass to 3.0% by mass, with respect to the total mass of the ceramide dispersion composition. If the content of the second surfactant is equal to or greater than 0.05% by mass with respect to the total mass of the ceramide dispersion composition, the dispersed particles tend to be atomized. If the content of the second surfactant is equal to or less than 8.0% by mass, the temporal stability of the ceramide dispersion composition tends to become excellent.

From the viewpoint of atomizing the dispersed particles and the temporal stability of the ceramide dispersion composition, the total content of the first surfactant and the second surfactant is preferably 0.5% by mass to 10.0% by mass, more preferably 0.7% by mass to 8.0% by mass, and even more preferably 1.0% by mass to 5.0% by mass, with respect to the total mass of the ceramide dispersion composition. If the total content of the first surfactant and the second surfactant is equal to or greater than 0.5% by mass with respect to the total mass of the ceramide dispersion composition, the dispersed particles tend to become fine particles. If the total content of the first surfactant and the second surfactant is equal to or less than 10.0% by mass, the temporal stability of the ceramide dispersion composition tens to become excellent.

The phospholipid excluded from the amphoteric surfactant which can be used as the second surfactant is a substance in which essential constituents thereof such as a glycerin skeleton, a fatty acid residue, and a phosphoric acid residue are bonded to a base, a polyol, or the like. Specific examples of phospholipid include various lecithins derived from plants such as soybean, corn, peanut, rape seeds, and barley, egg yolk, animals such as cows, and microorganisms such as E. coli; hydrogenated lecithin, enzymatically decomposed lecithin, enzymatically decomposed hydrogenated lecithin, and hydroxy lecithin, phosphatidic acid, phosphatidylglycerin, phosphatidylinositol, phosphatidyl ethanolamine, phosphatidyl methylethanolamine, phosphatidylcholine, phosphaditylserine, bis-phosphatidic acid, diphosphatidylglycerin (cardiolipin), sphingomyelin, and the like.

<Other components>

As long as the effects of the present invention are not impaired, the ceramide dispersion composition can contain other components, which can be generally added to an emulsified composition or a dispersion composition, in addition to the aforementioned components. These other components can be formulated as an aqueous phase composition or an oil phase composition of the ceramide dispersion composition. According to the purpose of the ceramide dispersion composition, for example, it is possible to concurrently use other additives generally used for this purpose, such as various medicinal components, a preservative, and a colorant.

Examples of those other additives include a cooling agent such as menthol or camphor, plant extracts, a pH buffering agent, an antioxidant, an ultraviolet absorber, an ultraviolet scattering agent, a preservative, a fragrance, a germicide, a colorant, and the like.

Examples of the pH adjuster include an organic acid, an organic base, an inorganic acid, an inorganic base, and a salt of these. Specific examples of the organic acid, the organic base, the inorganic acid, the inorganic base, and a salt of these include an organic acid and a salt thereof such as citric acid, ascorbic acid, glycolic acid, tartaric acid, succinic acid, acetic acid, phthalic acid, trifluoroacetic acid, morpholinoethanesulfonic acid, and 2-(4-(2-hydroxyethyl)-l-piperazinyl)ethanesulfonic acid; an organic base and a salt thereof such as tris(hydroxymethyl)aminomethane, and ammonia; an inorganic acid and a salt thereof such as hydrochloric acid, perchloric acid, carbonic acid, and phosphoric acid; an inorganic base and a salt thereof such as sodium phosphate, potassium phosphate, calcium hydroxide, sodium hydroxide, potassium hydroxide, and magnesium hydroxide; an inorganic hydrogen salt such as sodium hydrogen phosphate and sodium hydrogen carbonate; and the like.

<pH>

The pH of the ceramide dispersion composition is equal to or less than 6.5. For example, in a case where the ceramide dispersion composition is used as a hair cosmetic such as a hair conditioner, the pH of the ceramide dispersion composition is more preferably equal to or less than 5.5, and even more preferably equal to or less than 4.5. From the viewpoint of skin irritation, the pH of the ceramide dispersion composition is preferably equal to or greater than 1.0, more preferably equal to or greater than 2.0, and even more preferably equal to or greater than 3.0.

<Particle size>

In the present invention, the dispersed particles (ceramide-containing particles) contained in the ceramide dispersion composition are preferably fine particles having an average particle size of, for example, less than 1 $\mu$m. For instance, from the viewpoint of preventing the precipitation of the particles that occurs with the passage of a long time and from the viewpoint of the permeability of the ceramide-containing particles into the epidermis, hair, or the like of a biological body and the transparency of the ceramide dispersion composition, the average particle size of the dispersed particles contained in the ceramide dispersion composition is preferably equal to or less than 100 nm, more preferably equal to or less than 80 nm, and even more preferably equal to or less than 50 nm. The lower limit of the average particle size of the ceramide-containing particles is not particularly limited. The average particle size of the ceramide-containing particles can be, for example, equal to or greater than 0.8 nm.

In the present invention, the average particle size of the ceramide-containing particles means a volume average particle size of the dispersed particles present in the ceramide dispersion composition. Because of the accuracy and the simplicity of measurement, the average particle size of the dispersed particles contained in the ceramide dispersion composition is measured by using a dynamic light scattering method.

Examples of commercially available measurement instruments using dynamic light scattering include a concentrated system particle size analyzer FPAR-1000 (OTSUKA ELECTRONICS CO., LTD.), Nanotrac UPA (NIKKISO CO., LTD.), Nanosizer (manufactured by Malvern Instruments Ltd), and the like. In the present invention, as the average particle size of the dispersed particles, a value measured at a temperature of 23°C by using a Nanotrac UPA (NIKKISO CO., LTD.) is adopted. The particle size of the dispersed particles in the present invention is measured by diluting a sample taken from the ceramide dispersion composition of the present invention with pure water such that the concentration of ceramide contained in the sample becomes 0.04% by mass. The particle size can be determined as a volume average particle size which is obtained by setting a refractive index of a sample to be 1.600 and a refractive index of a dispersion medium to be 1.333 (pure water) and by using the viscosity of pure water as the viscosity of the dispersion medium.

Specifically, the ceramide dispersion composition used as a measurement sample is diluted 10 times with Milli-Q water, and the particle size of the dispersed particles is measured and determined as a volume average particle size (Mv).

The average particle size of the dispersed particles can be adjusted not only by the components of the ceramide dispersion composition but also by stirring conditions (shear force, temperature, or pressure) in a manufacturing method of the composition and a factor such as a ratio between an oil phase and an aqueous phase.

<Manufacturing method of ceramide dispersion composition>

The ceramide dispersion composition can be obtained by a manufacturing method including a step of mixing an oil phase (dispersed phase) component containing at least a natural ceramide with an aqueous phase (continuous phase) component.

For mixing the aqueous phase component with the oil phase component, a known method such as a high-pressure emulsification method in which a shear force of equal to or greater than 100 MPa is applied or a jet injection method in which the oil phase component is directly injected into the aqueous phase component may be used.

From the viewpoint of the particle size of the particles (dispersed particles) containing ceramide, the dispersion stability, and the preservation stability, as the method for mixing the aqueous phase component with the oil phase component, it is preferable to use a method using a micromixer in which the oil phase component and the aqueous phase component are independently passed through a micro-flow path, in which the narrowest portion has a cross-sectional area of 1 $\mu m^2$ to 1 $mm^2$, and then combined and mixed with each other. When the method using a micromixer is used, from the viewpoint of atomizing the dispersed particles, the viscosity of the aqueous phase is preferably equal to or less than 30 mPa·s.

Examples of the manufacturing method of the ceramide dispersion composition include a method including a step of mixing an aqueous phase component containing a polyol with an oil phase component containing at least a natural ceramide and a step of dispersing the mixture by using a high-pressure homogenizer or the like so as to obtain a ceramide dispersion composition (emulsion) containing ceramide-containing particles (dispersed particles). The first surfactant and the second surfactant may be formulated as either the aqueous phase component or the oil phase component.

A ratio (in terms of mass) between the oil phase and the aqueous phase at the time of dispersion is not particularly limited, but is preferably 0.05/99.95 to 50/50, more preferably 0.1/99.9 to 30/70, and even more preferably 0.5/99.5 to 20/80, in terms of ratio (mass %) of oil phase/aqueous phase. It is preferable that the ratio of oil phase/aqueous phase is within the above range, because then a sufficient amount of active components can be contained in the ceramide dispersion composition, and sufficient dispersion stability for practical use can be obtained.

At the time of mixing the oil phase component with the aqueous phase component and dispersing the mixture, it is preferable to obtain a crude dispersion by mixing the oil phase component with the aqueous phase component and then atomizing the crude dispersion by using atomization means.

As the means for obtaining the crude dispersion by mixing the oil phase component with the aqueous phase component, any of commercially available stirring means may be used. For example, by mixing and stirring together the oil phase component and the aqueous phase component in an aqueous medium by using a magnetic stirrer, a household mixer, a paddle mixer, an impeller mixer, or the like, a homogeneous crude dispersion can be prepared. As the means for obtaining the crude dispersion, it is more preferable to use stirring means having a strong shear force. That is, it is preferable to use a homomixer, a disper mixer, an ultramixer, or the like for mixing the oil phase component with the aqueous phase component.

In order to further improve the effect of the crude dispersion processing, it is also preferable to use ultrasonic waves in addition to these stirring means. As means for applying ultrasonic waves, it is preferable to use an ultrasonic homogenizer. Examples of the ultrasonic homogenizer include ultrasonic homogenizers US-600, US-1200T, RUS-1200T, and MUS-1200T (all manufactured by NISSEI Corporation.), ultrasonic processors UIP 2000, UIP-4000, UIP-8000, and UIP-16000 (all manufactured by Hielscher Ultrasonic GmbH), and the like. These high-power ultrasonic irradiation devices can be used at a frequency of equal to or less than 25 kHz and preferably at a frequency of 15 kHz to 20 kHz.

The crude dispersion processing for obtaining the crude dispersion by mixing the oil phase component with the aqueous phase component can be performed at any temperature within a temperature range of equal to or higher than 20°C and equal to or lower than 90°C. However, for example, the processing is preferably performed at a temperature of equal to or higher than 40°C and equal to or lower than 80°C.

It is preferable that the obtained crude dispersion is then atomized by using atomization and emulsification means.

As the atomization means, it is preferable to use a high-pressure homogenizer. Examples of the high-pressure homogenizer include an Ultimizer HJP-25005 (manufactured by SUGINO MACHINE LIMITED).

From the viewpoint of the dispersibility (atomization), the pressure at the time of manufacturing the ceramide dispersion composition is equal to or greater than 100 MPa and preferably equal to or greater than 150 MPa. From the viewpoint of the temperature increase and the pressure resistance, in commercially available products, the highest pressure limit is preferably equal to or less than 300 MPa. When the crude dispersion is atomized by using the atomization and emulsification means, the high-pressure processing may be performed once. However, in order to improve the homogeneity of the entire liquid, the high-pressure processing is preferably performed twice or more, and more preferably performed two times to five times.

The temperature before the high-pressure dispersion processing is preferably set to be 20°C to 80°C, and more preferably set to be 40°C to 70°C. It is preferable to rapidly cool the crude dispersion having undergone the high-pressure dispersion processing by using cooling means immediately after the high-pressure dispersion processing such that the temperature thereof is reduced and becomes equal to or lower than a predetermined temperature. As the cooling device, any of commercially available heat exchangers can be used.

<Temporal stability>

The ceramide dispersion composition of the present invention exhibits excellent temporal stability and allows the just prepared dispersed particles to remain as fine particles over a long period of time. In the present invention, the "temporal stability" means a property in which the particle size of the dispersed particles contained in the just prepared ceramide dispersion composition changes little over time. The temporal stability of the ceramide dispersion composition can be evaluated by comparing the particle size of the just prepared dispersed particles with the particle size of the dispersed particles in the ceramide dispersion composition after a predetermined period of time. In the ceramide dispersion composition, a difference between the particle size of the just prepared dispersed particles and the particle size of the dispersed particles after being stored for two weeks in a thermostatic bath at a temperature of 50°C is preferably equal to or less than 20 nm, more preferably equal to or less than 10 nm, and even more preferably equal to or less than 7 nm.

<Application>

The ceramide dispersion composition of the present invention can be formed as a dispersion composition containing fine dispersed particles which brings about an excellent emollient effect resulting from a natural ceramide. Therefore, the ceramide dispersion composition of the present invention is preferably used as is or as a constituent material in various applications utilizing the functions of a natural ceramide.

For example, the ceramide dispersion composition of the present invention can be widely used in pharmaceutical products (an external preparation and a skin preparation), cosmetics, cleansing agents, and the like. Examples of the pharmaceutical products include parenteral agents such as an ointment (an external preparation for skin or the like). Examples of the cosmetics include a toner, a serum, a gel, an emulsion, a hair conditioner, a hair treatment, a rinse, and the like. Examples of the cleansing agents include a facial cleanser, a body soap, a shampoo, and the like. However, the use of the ceramide dispersion composition of the present invention is not limited to the above.

When the ceramide dispersion composition of the present invention is used in pharmaceutical products such as an external preparation for skin or in cosmetics, if necessary, components that can be added to the pharmaceutical products and the cosmetics can be appropriately added thereto.

The ceramide dispersion composition of the present invention may be embodied to have any of general forms known as cosmetics such as a toner, a serum, a gel, an emulsion, a hair conditioner, a hair treatment, and a rinse.

The ceramide dispersion composition of the present invention contains fine dispersed particles and exhibits excellent temporal stability in an acidic condition. Therefore, the ceramide dispersion composition of the present invention is preferably used in a hair cosmetic. That is, a hair cosmetic of the present invention contains the ceramide dispersion composition of the present invention. The hair cosmetic can contain other components generally used in hair cosmetics, in addition to the ceramide dispersion composition of the present invention. Examples

Hereinafter, the present invention will be specifically described by using examples, but the present invention is not limited thereto. Herein, unless otherwise specified, a "part" is based on mass.

[Example 1]

The following components were stirred for 60 minutes at a temperature of 70°C, thereby preparing a crude dispersion.

| | |
|---|---|
| ·4% by mass ceramide liquid dispersion | 10.00 parts |
| ·60% by mass N-[3-alkyl(12,14)oxy-2-hydroxypropyl]-L-arginine hydrochloride solution | 0.50 parts |
| ·Decaglyceryl myristate (HLB = 14) | 0.20 parts |
| ·Glycerin | 35.00 parts |
| ·Citric acid | 0.3 9 parts |
| ·Citric acid dihydrate | 0.87 parts |
| ·Pure water | 53.00 parts |

The details of each of the components used for preparing the crude dispersion are as described later.

The obtained crude dispersion was dispersed for 4 minutes by using an ultrasonic homogenizer US-600 (manufactured by NISSEI Corporation.), thereby obtaining a preliminary dispersion.

Thereafter, the obtained preliminary dispersion was cooled to about 60°C and subjected to high-pressure emulsification (dispersion) at a pressure of 245 MPa by using an Ultimizer HJP-25005 (manufactured by SUGINO MACHINE LIMITED).

Subsequently, the obtained dispersion composition was filtered through a microfilter having an average pore size of 1 $\mu$m, thereby obtaining a ceramide dispersion composition. The pH of the obtained ceramide dispersion composition was 3.8.

[Examples 2 to 15, Reference Example 16 and Comparative examples 1 to 6]

Ceramide dispersion compositions of Examples 2 to 15, Reference Example 16 and Comparative examples 1 to 6 were obtained in the same manner as in Example 1, except that the components were formulated as described in Tables 1 to 3.

The numerical values relating to the formulation of the components shown in Tables 1 to 3 means parts by mass. Furthermore, "-" means that the corresponding component was not formulated, and "1,3-BG" means 1,3-butylene glycol.

The components described in Tables 1 to 3 are as follows.

As a 4% by mass ceramide liquid dispersion, a 4% by mass liquid dispersion of Liquid Crysta Ceramide 2 (Takasago Aromas Corporation) containing ceramide 2 was used.

As a 60% by mass N-[3-alkyl(12,14)oxy-2-hydroxypropyl]-L-arginine hydrochloride solution, Amisafe LMA-60 (Ajinomoto Co., Inc.) was used.

As decaglyceryl myristate, Decaglyn 1-M (HLB = 14.0, Nikko Chemicals Co., Ltd.) was used.

As sucrose stearate, Ryoto Sugar Ester S-1570 (HLB = 15, Mitsubishi-Kagaku Foods Corporation) was used.

As glycerin, concentrated cosmetic glycerin (Kao Corporation.) was used.

As 1,3-butylene glycol, citric acid, and citric acid dihydrate, those manufactured by Wako Pure Chemical Industries, Ltd. were used.

As stearamidopropyl dimethylamine, amidoamine MPS (Nikko Chemicals Co., Ltd.) was used.

As cocobetaine, Genegen B 1566 (Clariant Japan K.K.) was used.

As steartrimonium chloride, NIKKOL CA-2465 (Nikko Chemicals Co., Ltd.) was used.

As lecithin, Lecion P (RIKEN VITAMIN Co. Ltd.) was used.

Evaluation

The temporal stability of the obtained ceramide dispersion compositions of Examples 1 to 15, Reference Example 16 and Comparative examples 1 to 6 was evaluated as below.

(1) Particle size of dispersed particles of ceramide dispersion composition

The particle size (volume average particle size) of the ceramide-containing particles (or oil drop-like dispersed particles containing the ceramide-containing particles) in the just prepared ceramide dispersion composition was measured by using a Nanotrac UPA (NIKKISO CO., LTD.). The volume average particle size was measured by diluting a sample, which was taken from the ceramide dispersion composition, with pure water such that the concentration of ceramide contained in the sample became 0.04% by mass. The volume average particle size was determined as a volume average particle size (Mv) obtained by setting a refractive index of the sample to be 1.600 and a refractive index of a dispersion medium to be 1.333 (pure water) and setting the viscosity of pure water as the viscosity of the dispersion medium. The results are shown in Tables 1 to 3.

Furthermore, each of the ceramide dispersion compositions was stored for two weeks in a thermostatic bath at a temperature of 50°C and then returned to 25°C, and the particle size of the ceramide-containing particles (or oil droplet-like dispersed particles containing the ceramide-containing particles) was measured in the same manner as described above. The results are shown in Tables 1 to 3.

(2) Evaluation of precipitation properties

Each of the ceramide dispersion compositions was put into a glass bottle, sealed, and stored for two weeks in a thermostatic bath at a temperature of 50°C. Thereafter, the presence or absence of a precipitate was evaluated by visual observation.

[Table 1]

| | | | Example 1 | Example 2 | Example 3 | Example 4 | Example 5 | Example 6 | Example 7 | Example 8 | Example 9 |
|---|---|---|---|---|---|---|---|---|---|---|---|
| Formulation | | 4% by mass ceramide liquid dispersion | 10.00 | 10.00 | 10.00 | 10.00 | 10.00 | 10.00 | 10.00 | 10.00 | 10.00 |
| | Surfactant component | | | | | | | | | | |
| | | First surfactant (nonionic surfactant) | | | | | | | | | |
| | | Decaglyceryl myristate | 0.20 | 0.40 | 1.00 | 2.00 | 2.30 | 1.00 | 1.00 | 1.00 | 1.00 |
| | | Sucrose stearic acid ester | | | | | | | | | |
| | | Second surfactant (amphoteric surfactant and cationic surfactant) | | | | | | | | | |
| | | 60% by mass N-[3-alkyl (12,14)oxy-2-hydroxypropyl]-L-arginine hydrochloride solution | 0.50 | 0.50 | 0.50 | 0.50 | 0.50 | 0.50 | - | - | - |
| | | Stearamidopropyl dimethyl amine | - | - | - | - | - | - | 0.30 | - | - |
| | | Cocobetaine | - | - | - | - | - | - | - | 0.30 | - |
| | | Steartrimonium chloride | - | - | - | - | - | - | - | - | 0.30 |
| | | Lecithin | - | - | - | - | - | - | - | - | - |
| | Polyol | | | | | | | | | | |
| | | Glycerin | 35.00 | 35.00 | 35.00 | 35.00 | 35.00 | | 35.00 | 35.00 | 35.00 |
| | | 1,3-BG | - | - | - | - | - | 35.00 | - | - | - |
| | Others | | | | | | | | | | |
| | | Citric acid | 0.39 | 0.39 | 0.39 | 0.39 | 0.39 | 0.39 | 0.39 | 0.39 | 0.39 |
| | | Citric acid dihydrate | 0.87 | 0.87 | 0.87 | 0.87 | 0.87 | 0.87 | 0.87 | 0.87 | 0.87 |
| | | Water | 53.0 | 52.8 | 52.2 | 51.2 | 50.9 | 52.2 | 52.4 | 52.4 | 52.4 |
| pH | | | 3.8 | 3.8 | 3.8 | 3.8 | 3.8 | 3.8 | 3.8 | 3.8 | 3.8 |

12

(continued)

|  | | Example 1 | Example 2 | Example 3 | Example 4 | Example 5 | Example 6 | Example 7 | Example 8 | Example 9 |
|---|---|---|---|---|---|---|---|---|---|---|
| Evaluation | Particle size immediately after preparation | 72 nm | 20 nm | 9 nm | 14 nm | 18 nm | 30 nm | 30 nm | 37 nm | 35 nm |
| | Particle size after 2 weeks at 50°C | 79 nm | 21 nm | 9 nm | 18 nm | 29 nm | 50 nm | 35 nm | 42 nm | 39 nm |
| | Precipitate after 2 weeks at 50°C | Absent | Absent | Absent | Absent | Absent | Absent | Absent | Absent | Absent |

[Table 2]

| | | | Example 10 | Example 11 | Example 12 | Example 13 | Example 14 | Example 15 | Reference Example 16 |
|---|---|---|---|---|---|---|---|---|---|
| Formulation | 4% by mass ceramide liquid dispersion | | 10.00 | 10.00 | 10.00 | 10.00 | 1000 | 10.00 | 10.00 |
| | Surfactant component | First surfactant (nonionic surfactant) | | | | | | | |
| | | Decaglyceryl myristate | 1.00 | 1.00 | 1.00 | 1.00 | 1.00 | 1.00 | |
| | | Sucrose stearic acid ester | | | | | | | 1.00 |
| | | Second surfactant (amphoteric surfactant and cationic surfactant) | | | | | | | |
| | | 60% By mass N-[3-alkyl(12,14)oxy-2-hydroxypropyl]-L-arginine hydrochloride solution | 0.50 | 0.50 | 0.50 | 0.50 | 0.08 | 1.33 | 0.50 |
| | | Stearamidopropyl dimethyl amine | - | - | - | - | - | - | - |
| | | Cocobetaine | - | - | - | - | - | - | - |
| | | Steartrimonium chloride | - | - | - | - | - | - | - |
| | | Lecithin | - | - | - | - | - | - | - |
| | Polyol | Glycerin | 1.00 | 2.00 | 50.00 | 60.00 | 35.00 | 35.00 | 35.00 |
| | | 1,3-BG | - | - | - | - | - | - | - |
| | Others | Citric acid | 0.39 | 0.39 | 0.39 | 0.39 | 0.39 | 0.39 | 0.39 |
| | | Citric acid dihydrate | 0.87 | 0.87 | 0.87 | 0.87 | 0.87 | 0.87 | 0.87 |
| | | Water | 86.2 | 85.2 | 37.2 | 27.2 | 52.7 | 51.4 | 52.2 |
| pH | | | 3.8 | 3.8 | 3.8 | 3.8 | 3.8 | 3.8 | 3.8 |
| Evaluation | Particle size immediately after preparation | | 71 nm | 28 nm | 11 nm | 16 nm | 65 nm | 17 nm | 80 nm |
| | Particle size after 2 weeks at 50°C | | 74 nm | 33 nm | 15 nm | 36 nm | 77 nm | 30 nm | 91 nm |
| | Precipitate after 2 weeks at 50°C | | Absent | Absent | Absent | Absent | Absent | Absent | Absent |

[Table 3]

| | | | Comparative example | Comparative example 2 | Comparative example 3 | Comparative example 4 | Comparative example 5 | Comparative example 6 |
|---|---|---|---|---|---|---|---|---|
| Formulation | 4% by mass ceramide liquid dispersion | | 10.00 | 10.00 | 10.00 | 10.00 | 10.00 | 10.00 |
| | Surfactant component | | | | | | | |
| | | First surfactant (nonionic surfactant) | | | | | | |
| | | Decaglyceryl myristate | 1.00 | 2.00 | 1.00 | 1.00 | - | 0.20 |
| | | Sucrose stearic acid ester | | | | | | |
| | | Second surfactant (amphoteric surfactant and cationic surfactant) | | | | | | |
| | | 60% by mass N-[3-alkyl(12,14)oxy-2-hydroxypropyl]-L-arginine hydrochloride solution | - | - | - | - | 0.50 | - |
| | | Stearamidopropyl dimethyl amine | - | - | - | - | - | - |
| | | Cocobetaine | - | - | - | - | - | - |
| | | Steartrimonium chloride | - | - | - | - | - | - |
| | | Lecithin | - | - | - | - | - | 0.50 |
| | Polyol | | | | | | | |
| | | Glycerin | - | - | 35.00 | 35.00 | 35.00 | 35.00 |
| | | 1,3-BG | - | - | - | - | - | - |
| | Others | | | | | | | |
| | | Citric acid | 0.39 | 0.39 | 0.39 | 0.39 | 0.39 | 0.39 |
| | | Citric acid dihydrate | 0.87 | 0.87 | 0.87 | 0.87 | 0.87 | 0.87 |
| | | Water | 87.7 | 86.7 | 52.7 | 52.7 | 53.2 | 53.0 |
| pH | | | 3.8 | 3.8 | 3.8 | 3.8 | 3.8 | 3.8 |
| Evaluation | Particle size immediately after preparation | | 230 nm | 186 nm | 170 nm | 220 nm | 140 nm | 135 nm |
| | Particle size after 2 weeks at 50°C | | 242 nm | 268 nm | 182 nm | 280 nm | 158 nm | 150 nm |
| | Precipitate after 2 weeks at 50°C | | Present | Present | Present | Present | Present | Present |

EP 3 028 691 B1

As shown in Tables 1 to 3, it was confirmed that even under conditions of a pH of equal to or less than 4.0, all of the ceramide dispersion compositions of Examples 1 to 15 and Reference Example 16 that were obtained by using a combination of a specific second surfactant and a nonionic surfactant as a first surfactant can retain dispersed particles having a small particle size over a long period time with excellent stability, in contrast to the ceramide dispersion compositions of Comparative examples 1 to 6.

It is expected that even under conditions of a pH of equal to or less than 4.0, the hair cosmetics containing the ceramide dispersion compositions of Examples 1 to 15 and Reference Example 16 will be able to retain dispersed particles having a small particle size over a long period of time with excellent stability, in contrast to the hair cosmetics containing the ceramide dispersion compositions of Comparative examples 1 to 6.

Therefore, according to the present invention, it is possible to provide a ceramide dispersion composition which contains fine dispersed particles and exhibits excellent temporal stability under acidic conditions.

Hereinafter, examples of the hair cosmetic using the ceramide dispersion composition of the present invention and reference examples of the external preparation for skin using the ceramide dispersion composition of the present invention will be described.

«Tomato extract, krill extract, and hematococcus algae extract»

The details of tomato extract, krill extract, and hematococcus algae extract used for preparing the hair cosmetics of examples and the external preparations for skin of comparative examples are as below.

·Tomato extract (lycopene content: 0.1% by mass, obtained by diluting 6% Lyc-O-Mato (trade name, manufactured by Sun Bright Co., Ltd.) with glycerin)
·Krill extract (astaxanthin content: 0.6% by mass)
·Hematococcus algae extract (astaxanthin content: 0.3% by mass, obtained by diluting ASTOTS-S (trade name, manufactured by Takedashiki Co., Ltd.) with glycerin)

[Example 17]

A shampoo having the following composition was prepared (a total amount of 100% by mass). The shampoo had a pH of 3.8.

| (Component) | (% by mass) |
| --- | --- |
| ·Sodium PEG coconut oil fatty acid amide MEA sulfate | 3.0 |
| ·Sodium cocoyl sarcosinate | 3.0 |
| ·Sodium benzoate | 0.4 |
| ·Hematococcus algae extract | 0.01 |
| ·Krill extract | 0.01 |
| ·Tomato extract | 0.01 |
| ·Cocamidopropyl betaine | 4.0 |
| ·Lauramide DEA | 3.0 |
| ·Glycol distearate | 1.5 |
| ·Polyquaternium-10 | 0.5 |
| ·Citric acid | 0.3 |
| ·Sodium citrate | Appropriate amount |
| ·Tocopherol | 0.05 |
| ·Water-soluble collagen | 1.0 |
| ·Hydrolyzed collagen | 1.0 |
| ·Ceramide dispersion composition (Example 3) | 1.0 |
| ·Purified water | Balance |

[Example 18]

A hair care essence having the following composition was prepared (a total amount of 100% by mass). The essence had a pH of 3.7.

| (Component) | (% by mass) |
|---|---|
| ·Citric acid | 0.4 |
| ·PEG-60 hydrogenated castor oil | 0.3 |
| ·1,3-Butylene glycol | 2.0 |
| ·Glycerin | 1.0 |
| ·Pantothenyl ethyl ether | 0.5 |
| ·Nicotinic acid amide | 0.3 |
| ·Glycyrrhetinic acid | 0.15 |
| ·Lecithin | 0.5 |
| ·Acetyl hydroxyproline | 1.0 |
| ·Hydrolyzed collagen | 1.0 |
| ·Water-soluble collagen | 1.0 |
| ·Hematococcus algae extract | 0.1 |
| ·Hop extract | 1.0 |
| ·Loquat leaf extract | 1.0 |
| ·Phenoxy ethanol | 0.3 |
| ·Methy lparaben | 0.1 |
| ·Sodium benzoate | 0.1 |
| ·Ceramide dispersion composition (Example 3) | 1.0 |
| ·Water | Balance |

[Example 19]

A conditioner having the following composition was prepared (a total amount of 100% by mass). The conditioner had a pH of 4.0.

| (Component) | (% by mass) |
|---|---|
| ·Behenyl alcohol (C22) | 3.0 |
| ·Stearyl alcohol (C20) | 3.0 |
| ·Cetanol (C16) | 1.5 |
| ·Steartrimonium chloride | 2.0 |
| ·Ethylhexyl palmitate | 11.0 |
| ·Polyquaternium-10 | 0.5 |
| ·Astaxanthin liquid | 0.01 |
| ·Acetyl hydroxyproline | 0.1 |
| ·Sodium hyaluronate | 0.1 |
| ·Water-soluble collagen | 0.1 |
| ·Dipotassium glycyrrhetinate | 0.1 |
| ·Citric acid | 0.1 |
| ·Tocopherol | 0.1 |
| ·Phenoxyethanol | 0.4 |
| ·Ceramide dispersion composition (Example 3) | 1.0 |
| ·Fragrance | Appropriate amount |
| ·Purified water | Balance |

[Reference Example 1]

A toner having the following composition was prepared (a total amount of 100% by mass)

| (Component) | (% by mass) |
|---|---|
| ·Glycerin | 5.0 |

(continued)

| (Component) | (% by mass) |
|---|---|
| ·1,3-Butylene glycol | 6.5 |
| ·Polyoxyethylene sorbitan monolauric acid ester | 1.2 |
| ·Hematococcus algae extract | 0.01 |
| ·Tomato extract | 0.01 |
| ·Ethanol | 6.0 |
| ·Tocopherol acetate | 0.001 |
| ·Vitamin E | 0.002 |
| ·Magnesium ascorbyl phosphate | 0.5 |
| ·Xanthan gum | 0.2 |
| ·Glycacil 2000 (*1) | 0.1 |
| ·Ethylhexyl glycerin | 0.2 |
| ·Pentylene glycol | 1.0 |
| ·Hydrolyzed collagen | 1.0 |
| ·N-Acetylhydroxyproline | 1.0 |
| ·Water-soluble collagen | 1.0 |
| ·Polyoxyethylene hydrogenated castor oil | 0.2 |
| ·Decaglyceryl monooleate | 0.05 |
| ·Ceramide dispersion composition (Example 3) | 1.0 |
| ·Fragrance | Appropriate amount |
| ·Purified water | Balance |
| *1: Glycacil 2000 (manufactured by Lonza Group Ltd.) contains iodopropynyl butylcarbamate and cyclodexitrin. | |

[Reference Example 2]

An emulsion having the following composition was prepared (a total amount of 100% by mass).

| (Component) | (% by mass) |
|---|---|
| ·Tomato extract | 0.1 |
| ·Hematococcus algae extract | 0.5 |
| ·Lutein | 0.01 |
| ·Squalane | 8.0 |
| ·Jojoba seed oil | 7.0 |
| ·Glyceryl p-aminobenzoate | 1.0 |
| ·Cetyl alcohol | 1.5 |
| ·Glycerin monostearate | 2.0 |
| ·Polyoxyethylene cetyl ether | 3.0 |
| ·Polyoxyethylene sorbitan monooleate | 2.0 |
| ·1,3-Butylene glycol | 1.0 |
| ·Glycerin | 2.0 |
| ·Methylparaben | 0.04 |
| ·Sucrose stearate | 0.1 |
| ·Polyglyceryl-10 oleate | 0.1 |
| ·Tocopherol acetate | 0.01 |
| ·Phenoxyethanol | 0.2 |
| ·Collagen | 1.0 |
| ·Sodium citrate | 1.0 |
| ·Ceramide dispersion composition (Example 3) | 1.0 |
| ·Fragrance | Appropriate amount |
| ·Purified water | Balance |

It was confirmed that even when fucoxanthin is used instead of lutein in the above composition, an emulsion can be prepared in the same manner.

[Reference Example 3]

A cream having the following composition was prepared (a total amount of 100% by mass).

| (Component) | (% by mass) |
|---|---|
| ·Tomato extract | 0.4 |
| ·Krill extract | 0.2 |
| ·Cetostearyl alcohol | 3.0 |
| ·Glycerin fatty acid ester | 2.0 |
| ·Polyoxyethylene (20) sorbitan monooleate | 1.0 |
| ·Sorbitan monostearate | 1.0 |
| ·Sodium N-stearoyl-N-methyltaurate | 0.5 |
| ·Vaseline | 5.0 |
| ·Lecithin | 0.5 |
| ·Dimethylpolysiloxane (100 mPa·s) | 3.0 |
| ·Glyceryl tri-2-ethylhexanoate | 20.0 |
| ·Lactic acid | 1.0 |
| ·Magnesium ascorbyl phosphate | 0.5 |
| ·Dipropylene glycol | 10.0 |
| ·Sodium citrate | 0.5 |
| ·Titanium oxide | 0.1 |
| ·Fragrance | Appropriate amount |
| ·Disodium edetate | 0.03 |
| ·Ethyl p-oxybenzoate | 0.05 |
| ·Ceramide dispersion composition (Example 3) | 1.0 |
| ·Purified water | Balance |

[Reference Example 4]

A jelly-type serum having the following composition was prepared by a common method (a total amount of 100% by mass)

| (Component) | (% by mass) |
|---|---|
| ·Tomato extract | 0.01 |
| ·Hematococcus algae extract | 0.02 |
| ·Tocopherol | 0.01 |
| ·Ceramides III and VI | 0.01 |
| ·(PEG-240/decyltetradeceth-20/HDI) copolymer | 0.5 |
| ·Hydrolyzed collagen | 1.0 |
| ·Acetyl hydroxyproline | 1.0 |
| ·Ethylhexyl glycerin | 0.05 |
| ·Oleic acid | 0.05 |
| ·1,3-Butylene glycol | 1.0 |
| ·Glycerin | 2.0 |
| ·Methylparaben | 0.2 |
| ·Sucrose stearate | 0.01 |
| ·Polyglyceryl-2 oleate | 0.01 |
| ·Polyglyceryl-2-stearate | 0.01 |
| ·Phenoxyethanol | 0.2 |
| ·Collagen | 1.0 |

(continued)

| (Component) | (% by mass) |
|---|---|
| ·Sodium citrate | 1.0 |
| ·Ceramide dispersion composition (Example 3) | 1.0 |
| ·Rosa damascena flower oil | Appropriate amount |
| ·Fragrance | Appropriate amount |
| ·Purified water | Balance |

[Reference Example 5]

A UV protector (water-in-oil type emulsion) having the following composition was prepared (a total amount of 100% by mass).

| (Component) | (% by mass) |
|---|---|
| ·Hematococcus algae extract | 0.025 |
| ·Tomato extract | 0.025 |
| ·Pterostilbene | 0.1 |
| ·Cyclopentasiloxane | 35.0 |
| ·t-Butylmethoxydibenzoylmethane-modified titanium oxide (*1) | 12.0 |
| ·PEG-9 polydimethylsiloxyethyl dimethicone (*2) | 7.5 |
| ·Ethanol | 5.0 |
| ·(Butyl acrylate/glycol dimethacrylate) cross-polymer | 2.0 |
| ·Sorbitan sesquioleate | 2.0 |
| ·Magnesium sulfate | 0.1 |
| ·(Dimethicone/(PEG-10/15)) cross-polymer | 1.0 |
| ·Mica | 0.5 |
| ·Phenoxy ethanol | 0.5 |
| ·(Dimethicone/vinyldimethicone) cross-polymer | 0.2 |
| ·(Methyl methacrylate/glycol dimethacrylate) cross-polymer | 0.1 |
| ·Iron oxide | 0.1 |
| ·Sodium hyaluronate | 1.0 |
| ·Rosa damascena flower oil | Appropriate amount |
| ·Hydrolyzed collagen | 1.0 |
| ·Acetyl hydroxyproline | 1.0 |
| ·Disodium stearoyl glutamate | 1.0 |
| ·Water-soluble collagen | 1.0 |
| ·Glyceryl tri(caprylate/caprate) | 0.1 |
| ·Polyglyceryl-10 oleate | 0.1 |
| ·Sucrose stearate | 0.1 |
| ·Lecithin | 0.1 |
| ·Tocopherol | 0.2 |
| ·Dipotassium glycyrrhizinate | 0.1 |
| ·Ceramide dispersion composition (Example 3) | 1.0 |
| ·Purified water | Balance |

*1: Hydrophobized powder HXMT-100 (TAYCA) composed of titanium oxide, t-butylmethoxydibenzoylmethane, aluminum hydroxide, and isostearic acid.
*2: KF-6028 (manufactured by Shin-Etsu Chemical Co., Ltd.)

[Reference Example 6]

Liquid oil having the following composition was prepared (a total amount of 100% by mass).

| (Component) | (% by mass) |
|---|---|
| ·Ethylhexyl palmitate | 35.0 |
| ·Glyceryl tri(caprylate/caprate) | 25.0 |
| ·Polyglyceryl-10 trilaurate | 25.0 |
| ·Diisostearyl malate | 10.0 |
| ·Tocopherol | 0.02 |
| ·Hematococcus algae extract | 0.01 |
| ·Tomato extract | 0.01 |
| ·Rosa damascena flower oil | Appropriate amount |
| ·N-Acetylhydroxyproline | 1.0 |
| ·Fragrance | Appropriate amount |
| ·Phenoxyethanol | 0.4 |
| ·Ceramide dispersion composition (Example 3) | 0.1 |
| ·Hydrogenated polyisobutene | Balance |

[Reference Example 7]

Moisturizing foam having the following composition was prepared (a total amount of 100% by mass).

| (Component) | (% by mass) |
|---|---|
| ·Myristic acid | 12.0 |
| ·Butylene glycol | 8.0 |
| ·Potassium hydroxide | 4.0 |
| ·Glycerin | 5.0 |
| ·Stearic acid | 8.0 |
| ·Sorbitol | 1.0 |
| ·Glyceryl stearate | 5.0 |
| ·Sodium lauroyl glutamate | 8.0 |
| ·(Lauramide/myristamide) DEA | 8.0 |
| ·Tocopherol | 0.1 |
| ·Water-soluble collagen | 1.0 |
| ·Hematococcus algae extract | 0.1 |
| ·Tomato extract | 0.1 |
| ·Rosa damascena flower oil | Appropriate amount |
| ·Shea butter | 2.0 |
| ·Jojoba seed oil | 2.0 |
| ·Polyquaternium-39 | 0.5 |
| ·Fatty acid (C10 to 30) (cholesteryl/lanosteryl) | 3.0 |
| ·Fragrance | Appropriate amount |
| ·Phenoxyethanol | 0.4 |
| ·Ceramide dispersion composition (Example 3) | 1.0 |
| ·Purified water | Balance |

[Reference Example 8]

A mascara having the following composition was prepared (a total amount of 100% by mass)

| (Component) | (% by mass) |
|---|---|
| ·Micro-crystalline wax | 5.0 |
| ·Carnauba wax | 5.0 |
| ·Beeswax | 5.0 |
| ·Paraffin wax | 5.0 |

(continued)

| (Component) | (% by mass) |
|---|---|
| ·Isostearic acid | 5.0 |
| ·Cetanol | 1.0 |
| ·Triethanolamine | 2.0 |
| ·1,3-Butylene glycol | 3.0 |
| ·Ethanol | 3.0 |
| ·Polyvinyl alcohol | 1.0 |
| ·Hydroxyethyl cellulose | 0.5 |
| ·Sodium polyacrylate | 0.1 |
| ·Black iron oxide | 2.0 |
| ·Carbon black | 2.0 |
| ·Nylon powder | 3.0 |
| ·Phenoxyethanol | 0.5 |
| ·Ceramide dispersion composition (Example 3) | 0.1 |
| ·Purified water | Balance |

[Reference Example 9]

A mascara having the following composition was prepared (a total amount of 100% by mass).

| (Component) | (% by mass) |
|---|---|
| ·Cyclopentasiloxane | 5.0 |
| ·Trimethylsiloxysilicate | 5.0 |
| ·Sorbitan sesquioleate | 2.0 |
| ·Polyethylene wax | 5.0 |
| ·Carnauba wax | 5.0 |
| ·Beeswax | 7.0 |
| ·Isostearic acid | 3.0 |
| ·Methylphenylpolysiloxane | 3.0 |
| ·Dextrin palmitate | 3.0 |
| ·Purified water | 3.0 |
| ·Acrylic acid-alkyl methacrylate copolymer emulsion (solid contents: 30%) | 15.0 |
| ·Vinyl acetate-vinyl pyrrolidone copolymer | 0.5 |
| ·Dipropylene glycol | 1.0 |
| ·1,3-Butylene glycol | 2.0 |
| ·Polyvinyl alcohol | 0.5 |
| ·Hydroxyethyl cellulose | 0.5 |
| ·Black iron oxide | 6.0 |
| ·Ceramide dispersion composition (Example 3) | 0.1 |
| ·Isoparaffin | Balance |

[Reference Example 10]

A lip balm having the following composition was prepared (a total amount of 100% by mass)

| (Component) | (% by mass) |
|---|---|
| ·Hydrogenated polyisobutene | 35.0 |
| ·Liquid paraffin | 25.0 |
| ·Diisostearyl malate | 20.0 |
| ·Dextrin palmitate | 5.0 |
| ·Polyglyceryl-2 triisostearate | 3.0 |

(continued)

| (Component) | (% by mass) |
|---|---|
| ·Vaseline | 2.0 |
| ·Tocopherol | 0.5 |
| ·Pearl pigment | 5.0 |
| ·Ceramide dispersion composition (Example 3) | 0.1 |
| ·Titanium oxide | Appropriate amount |
| ·Fragrance | Appropriate amount |
| ·Preservative | Appropriate amount |

[Reference Example 11]

An emulsion-type foundation having the following composition was prepared (a total amount of 100% by mass).

| (Component) | (% by mass) |
|---|---|
| ·Cyclohexasiloxane | 24.0 |
| ·Squalane | 2.0 |
| ·Isotridecyl isononanoate | 5.0 |
| ·Polyglyceryl-2 diisostearate | 2.0 |
| ·PEG-10 dimethicone | 5.0 |
| ·Tocopherol | 0.5 |
| ·Phenoxyethanol | 0.4 |
| ·Quaternium-18 bentonite | 2.0 |
| ·Titanium oxide | 18.0 |
| ·Disodium EDTA | 0.2 |
| ·1,3-Butylene glycol | 5.0 |
| ·Ceramide dispersion composition (Example 3) | 0.1 |
| ·Black iron oxide | Appropriate amount |
| ·Red iron oxide | Appropriate amount |
| ·Yellow iron oxide | Appropriate amount |
| ·Purified water | Balance |

[Reference Example 12]

An emulsion-type foundation having the following composition was prepared (a total amount of 100% by mass).

| (Component) | (% by mass) |
|---|---|
| ·Polyglyceryl-2 sesquiisostearate | 2.5 |
| ·Titanium oxide | 8.0 |
| ·Beeswax | 2.5 |
| ·Cyclopentasiloxane | 7.0 |
| ·Lauryl PEG-9 polydimethylsiloxane ethyl dimethicone | 5.0 |
| ·Black iron oxide | 0.2 |
| ·Red iron oxide | 0.3 |
| ·Yellow iron oxide | 0.9 |
| ·Talc | 6.0 |
| ·NaCl | 2.0 |
| ·Ceramide dispersion composition (Example 3) | 0.1 |
| ·(Dimethicone/vinyl dimethicone) cross-polymer | 0.5 |
| (Dimethicone/(PEG-10/15)) cross-polymer | 0.5 |
| ·Purified water | Balance |

**Claims**

1. A ceramide dispersion composition having a pH of equal to or less than 6.5, comprising:

   a natural ceramide;
   a polyol;
   a first surfactant which is a nonionic surfactant; and
   a second surfactant which is at least one selected from the group consisting of a cationic surfactant and an amphoteric surfactant excluding phospholipids,
   wherein the first surfactant comprises a polyglyceryl fatty acid.

2. The ceramide dispersion composition according to claim 1,
   wherein the second surfactant comprises at least one component selected from the group consisting of an amino acid derivative, a quaternary ammonium salt, and an amidoamine derivative.

3. The ceramide dispersion composition according to claim 1 or 2,
   wherein the second surfactant comprises an arginine derivative.

4. The ceramide dispersion composition according to any one of claims 1 to 3,
   wherein an oil phase component which comprises at least the natural ceramide is in a form of particles having an average particle size equal to or less than 100 nm and being dispersed in an aqueous phase component.

5. The ceramide dispersion composition according to any one of claims 1 to 4,
   wherein the polyol comprises glycerin.

6. The ceramide dispersion composition according to any one of claims 1 to 5,
   wherein the natural ceramide comprises ceramide 2.

7. The ceramide dispersion composition according to any one of claims 1 to 6,
   wherein a content of the first surfactant is from 0.2% by mass to 2.5% by mass with respect to a total mass of the ceramide dispersion composition.

8. The ceramide dispersion composition according to any one of claims 1 to 7,
   wherein a content of the polyol is from 2.0% by mass to 50.0% by mass with respect to a total mass of the ceramide dispersion composition.

9. The ceramide dispersion composition according to any one of claims 1 to 8,
   wherein a content of the second surfactant is from 0.05% by mass to 8.0% by mass with respect to a total mass of the ceramide dispersion composition.

10. The ceramide dispersion composition according to any one of claims 1 to 9,
    wherein a total content of the first surfactant and the second surfactant is from 0.5% by mass to 10.0% by mass with respect to a total mass of the ceramide dispersion composition.

11. The ceramide dispersion composition according to any one of claims 1 to 10, wherein a total content of the first surfactant and the second surfactant is from 0.7% by mass to 5.0% by mass with respect to a total mass of the ceramide dispersion composition.

12. The ceramide dispersion composition according to any one of claims 1 to 11, wherein a content of the natural ceramide is from 0.1% by mass to 5.0% by mass with respect to a total mass of the ceramide dispersion composition.

13. A hair cosmetic comprising the ceramide dispersion composition according to any one of claims 1 to 12.

**Patentansprüche**

1. Ceramid-Dispersionszusammensetzung mit einem pH-Wert von gleich oder weniger als 6,5, umfassend:

ein natürliches Ceramid;

ein Polyol;

ein erstes Tensid, das ein nichtionisches Tensid ist; und

ein zweites Tensid, das mindestens eines, ausgewählt aus der Gruppe bestehend aus einem kationischen Tensid und einem amphoteren Tensid mit Ausnahme von Phospholipiden ist,

wobei das erste Tensid eine Polyglycerylfettsäure umfasst.

2. Ceramid-Dispersionszusammensetzung nach Anspruch 1,

wobei das zweite Tensid mindestens eine Komponente, ausgewählt aus der Gruppe bestehend aus einem Aminosäurederivat, einem quaternären Ammoniumsalz und einem Amidoaminderivat umfasst.

3. Ceramid-Dispersionszusammensetzung nach Anspruch 1 oder 2,

wobei das zweite Tensid ein Argininderivat umfasst.

4. Ceramid-Dispersionszusammensetzung nach einem der Ansprüche 1 bis 3,

wobei eine Ölphasenkomponente, die zumindest das natürliche Ceramid umfasst, in einer Form von Partikeln mit einer durchschnittlichen Partikelgröße von gleich oder weniger als 100 nm und dispergiert in einer Wasserphasenkomponente vorliegt.

5. Ceramid-Dispersionszusammensetzung nach einem der Ansprüche 1 bis 4,

wobei das Polyol Glycerin umfasst.

6. Ceramid-Dispersionszusammensetzung nach einem der Ansprüche 1 bis 5,

wobei das natürliche Ceramid Ceramid 2 umfasst.

7. Ceramid-Dispersionszusammensetzung nach einem der Ansprüche 1 bis 6,

wobei ein Gehalt an dem ersten Tensid von 0,2 Masse% bis 2,5 Masse%, bezogen auf eine Gesamtmasse der Ceramid-Dispersionszusammensetzung, beträgt.

8. Ceramid-Dispersionszusammensetzung nach einem der Ansprüche 1 bis 7,

wobei ein Gehalt an dem Polyol von 2,0 Masse% bis 50,0 Masse%, bezogen auf eine Gesamtmasse der Ceramid-Dispersionszusammensetzung, beträgt.

9. Ceramid-Dispersionszusammensetzung nach einem der Ansprüche 1 bis 8,

wobei ein Gehalt an dem zweiten Tensid von 0,05 Masse% bis 8,0 Masse%, bezogen auf eine Gesamtmasse der Ceramid-Dispersionszusammensetzung, beträgt.

10. Ceramid-Dispersionszusammensetzung nach einem der Ansprüche 1 bis 9,

wobei ein Gesamtgehalt an dem ersten Tensid und dem zweiten Tensid von 0,5 Masse% bis 10,0 Masse%, bezogen auf eine Gesamtmasse der Ceramid-Dispersionszusammensetzung, beträgt.

11. Ceramid-Dispersionszusammensetzung nach einem der Ansprüche 1 bis 10, wobei ein Gesamtgehalt an dem ersten Tensid und dem zweiten Tensid von 0,7 Masse% bis 5,0 Masse%, bezogen auf eine Gesamtmasse der Ceramid-Dispersionszusammensetzung, beträgt.

12. Ceramid-Dispersionszusammensetzung nach einem der Ansprüche 1 bis 11, wobei ein Gehalt an dem natürlichen Ceramid von 0,1 Masse% bis 5,0 Masse%, bezogen auf eine Gesamtmasse der Ceramid-Dispersionszusammensetzung, beträgt.

13. Haarkosmetik umfassend die Ceramid-Dispersionszusammensetzung nach einem der Ansprüche 1 bis 12.

**Revendications**

1. Composition de dispersion de céramide présentant un pH inférieur ou égal à 6,5, comprenant :

un céramide naturel ;

un polyol ;

un premier agent tensio-actif, lequel est un agent tensio-actif non ionique, et
un second agent tensio-actif, lequel est au moins un sélectionné parmi le groupe consistant en un agent tensio-actif cationique et un agent tensio-actif amphotère excluant les phospholipides,
dans laquelle le premier agent tensio-actif comprend un acide gras de polyglycéryle.

2. Composition de dispersion de céramide selon la revendication 1,
dans laquelle le second agent tensio-actif comprend au moins un composant sélectionné parmi le groupe consistant en un dérivé d'acide aminé, un sel d'ammonium quaternaire, et un dérivé amidoamine.

3. Composition de dispersion de céramide selon la revendication 1 ou 2,
dans laquelle le second agent tensio-actif comprend un dérivé arginine.

4. Composition de dispersion de céramide selon l'une quelconque des revendications 1 à 3,
dans laquelle un composant de phase huileuse, lequel comprend au moins le céramide naturel se présente sous la forme de particules présentant une taille de particules moyenne inférieure ou égale à 100 nm et dispersées dans un composant de phase aqueuse.

5. Composition de dispersion de céramide selon l'une quelconque des revendications 1 à 4,
dans laquelle le polyol comprend de la glycérine.

6. Composition de dispersion de céramide selon l'une quelconque des revendications 1 à 5,
dans laquelle le céramide naturel comprend un céramide 2.

7. Composition de dispersion de céramide selon l'une quelconque des revendications 1 à 6,
dans laquelle une teneur du premier agent tensio-actif s'étend de 0,2 % en masse à 2,5 % en masse par rapport à une masse totale de la composition de dispersion de céramide.

8. Composition de dispersion de céramide selon l'une quelconque des revendications 1 à 7,
dans laquelle une teneur du polyol s'étend de 2,0 % en masse à 50,0 % en masse par rapport à une masse totale de la composition de dispersion de céramide.

9. Composition de dispersion de céramide selon l'une quelconque des revendications 1 à 8,
dans laquelle une teneur du second agent tensio-actif s'étend de 0,05 % en masse à 8,0 % en masse par rapport à une masse totale de la composition de dispersion de céramide.

10. Composition de dispersion de céramide selon l'une quelconque des revendications 1 à 9,
dans laquelle une teneur totale du premier agent tensio-actif et du second tensio-actif s'étend de 0,5 % en masse à 10,0 % en masse par rapport à une masse totale de la composition de dispersion de céramide.

11. Composition de dispersion de céramide selon l'une quelconque des revendications 1 à 10,
dans laquelle une teneur totale du premier agent tensio-actif et du second agent tensio-actif s'étend de 0,7 % en masse à 5,0 % en masse par rapport à une masse totale de la composition de dispersion de céramide.

12. Composition de dispersion de céramide selon l'une quelconque des revendications 1 à 11,
dans laquelle une teneur du céramide naturel s'étend de 0,1 % en masse à 5,0 % en masse par rapport à une masse totale de la composition de dispersion de céramide.

13. Cosmétique capillaire comprenant la composition de dispersion de céramide selon l'une quelconque des revendications 1 à 12.

**EP 3 028 691 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010090092 A
- WO 2009145299 A
- JP 2008069108 A
- JP 2007001950 A
- JP 2003300842 A
- EP 2452668 A1
- US 2005287095 A1
- JP 2003113393 A